# EUROPEAN PATENT SPECIFICATION

(11) **EP 2 958 531 B1**
(45) Date of publication and mention of the grant of the patent: **07.09.2016**
(21) Application number: 14725700.0
(22) Date of filing: 22.05.2014
(51) Int. Cl.: A61F 9/008

(54) **TECHNIQUE FOR SETTING ENERGY-RELATED LASER-PULSE PARAMETERS**
VERFAHREN ZUR EINSTELLUNG ENERGIEBEZOGENER LASERIMPULSPARAMETER
TECHNIQUE POUR RÉGLER DES PARAMÈTRES D'IMPULSION LASER ASSOCIÉS À L'ÉNERGIE

(43) Date of publication of application: 30.12.2015
(73) Proprietor: WaveLight GmbH, 91058 Erlangen (DE)
(72) Inventor: FOESEL, Matthias, 96117 Memmelsdorf (DE); DONITZKY, Christof, 90542 Eckental/Eschenau (DE); VOGLER, Klaus, 99444 Blankenhain (DE); KITTELMANN, Olaf, 14109 Berlin (DE)
(74) Representative: Katérle, Axel
(86) International application number: PCT/EP2014/060565
(87) International publication number: WO 2015/176759

(56) References cited:
- WO-A1-2013/152875
- US-A- 5 656 186

## Description

### Technical Field

The present disclosure relates to setting laser-pulse parameters. It relates in particular to techniques for setting energy-related laser-pulse parameters.

### Background

Especially in industrial and medical environments, pulsed focused laser radiation has become an important means for material processing. In typical applications of pulsed laser radiation, electromagnetic and/or thermal effects of absorbed laser radiation are used to locally change or disrupt a target material in an irradiated region thereof in order to create a cut in, or ablate matter from, the target. Focusing the incident radiation allows for an increased local intensity of the radiation and for a spatially closer confined zone of interaction with the target material. In addition, the use of pulsed radiation instead of continuous radiation reduces the effect of heat accumulation in the treated object.

In medical laser applications, e.g., in laser-assisted eye surgery including, but not limited to, LASIK (Laser in-situ Keratomileusis), Keratoplasty, refractive lenticule extraction, etc., and also in other types of material processing using laser radiation, a sharply defined scope of the laser treatment and a low total transfer of energy into the treated material are usually desired. For that purpose, the use of ultra-short laser pulses having a pulse width in the range below one picosecond has been suggested. Conventionally used pulse durations may be anywhere between 250 fs and 800 fs, for example. At the same time, it is attempted to set the energy of each single pulse as small as possible, i.e., close to a threshold energy for achieving disruption or any other desired effect in the target.

When adjusting required pulse energies to different pulse durations for reaching a desired effect, it has been observed that, although shorter pulses may have a higher threshold power, the product of threshold power and pulse duration, i.e., the threshold pulse energy, often decreases for shorter pulse durations. However, it is known in this regard that for various applications there exist characteristic pulse widths below which the threshold pulse energy can be observed to increase again. In that case, given that a minimal transfer of energy is desired, ideal pulse lengths could be determined, which may only vary for different applications and different beam or target parameters. Corresponding techniques have been described, e.g., in the US 5 656 186 A.

Ideal laser pulse characteristics depend substantially on the treated material and the intended effect of a laser treatment. For example, in many practical applications a modification of the pulse length may become desirable in connection with a change of the process. Problems may arise when the settings of energy-related pulse parameters that were tuned to fit a particular pulse length become suboptimal in terms of a minimal energy transfer when the pulse length is changed. At the same time, a user of a laser apparatus may not be able to easily identify, e.g., during processing, the ideal pulse energy for a chosen pulse length and thus may unduly stress the target by exposing it to radiation high above the required energy level.

This has again particular disadvantages in surgery, especially in laser eye surgery, when cuts under a surface of the eye are performed. For, in such cases excessive pulse energy often leads to undesirably large vapor bubbles within the eye tissue in consequence of evaporated eye tissue; the size of the bubbles may be substantially larger than the focus diameter of the laser beam itself. Such bubbles stress the surrounding eye tissue and they change the optical properties of the operation zone such that the laser process itself, or related optical techniques, are negatively affected. Moreover, if in the described scenario a series of pulses is directed in close proximity to one another, the produced bubbles can connect to even larger cells and thus enhance their negative effects. While several techniques for removing such gas volumes are known, it would be beneficial if, for a range of different applications, their occurrence could be held at a minimum. This, however, requires an adaptation of laser parameters with a change of the application.

Techniques related to configuring radiation parameters for laser eye surgery have been described, e.g., in the WO 2013/152875 A1.

Still, a technique for facilitated setting of laser pulse parameters is desirable.

### Summary

According to a first aspect a method for energy setting of pulsed, focused laser radiation according to claim 1 is described. The method comprises the steps of establishing a relationship between a threshold pulse energy required for causing irreversible damage in a material and a pulse duration, the relationship allowing to obtain a threshold pulse energy for each of a plurality of pulse durations, the plurality of pulse durations including one or more pulse durations in a range between 200 fs and smaller; for a given pulse duration in the range between 200 fs and smaller, determining an associated threshold pulse energy based on the established relationship; and setting the pulse energy of the laser radiation based on the determined associated threshold pulse energy, wherein the relationship defines a decreasing threshold pulse energy for a decreasing pulse duration in the range between 200 fs and smaller.

The relationship may represent a decrease of the threshold pulse energy substantially as a function of the cubic root of the pulse duration. In certain embodiments, the function is a linear function of the cubic root of the pulse duration. In addition or as an alternative, the relationship may define the threshold pulse energy as a value of at most 0.35 µJ, e.g., at most 0.30 µJ or at most 0.25 µJ or at most 0.20 µJ or at most 0.15 µJ, for a pulse duration of 300 fs or smaller. In addition or as an alternative, the relationship may define the threshold pulse energy as a value in the range from 0.15 µJ to 0.30 µJ, e.g., in the range from 0.15 µJ to 0.20 µJ or from 0.20 µJ to 0.25 µJ or from 0.25 µJ to 0.30 µJ or from 0.20 µJ to 0.30 µJ, for a pulse duration of 200 fs. In addition or as an alternative, the relationship may define the threshold pulse energy as a value in the range from 0.05 µJ to 0.10 µJ, e.g., in the range from 0.05 µJ to 0.08 µJ or from 0.08 µJ to 0.10 µJ, for a pulse duration of 10 fs.

The establishing step may include the steps of irradiating, for each of a plurality of reference pulse durations above 200 fs, an object with a series of pulses of the laser radiation to create a damage site for each pulse of the series, wherein the pulse energy is set differently for each pulse of the series, determining a size of each damage site, determining a reference threshold pulse energy for each of the plurality of reference pulse durations based on the determined sizes of the damage sites created at the respective reference pulse duration, and determining the relationship based on the determined reference threshold pulse energies.

Each reference threshold pulse energy may be determined based on an extrapolation to zero size of the determined sizes of the damage sites created at the respective reference pulse duration. The sizes may be determined, for example, based on a diameter, an area or a volume of each damage site. The extrapolation may be based, for example, on a linear, an exponential, or a polynomial fit or any combination thereof applied to the determined sizes.

In addition or as an alternative, determining the relationship may include determining a linear approximation of the threshold pulse energy in dependence on the pulse duration.

The relationship may be established for a focus diameter of the laser radiation of no more than 10 µm or 7 µm or 5 µm, wherein the focus diameter represents the diameter of a pulse portion containing 86 % of the energy of a pulse of the radiation.

The damage may include a photodisruption caused by a laser-induced optical breakdown of the material.

The method may include the step of directing the laser radiation having the set pulse energy at a non-biological material or a biological material to create an incision in the material. The material may be human eye tissue.

According to a second aspect a method for fluence setting of pulsed, focused laser radiation according to claim 6 is described. The method comprises the steps of establishing a relationship between a threshold pulse fluence required for causing irreversible damage in a material and a pulse duration, the relationship allowing to obtain a threshold pulse fluence for each of a plurality of pulse durations, the plurality of pulse durations including one or more pulse durations in a range between 200 fs and smaller; for a given pulse duration in the range between 200 fs and smaller, determining an associated threshold pulse fluence based on the established relationship; and setting the pulse fluence of the laser radiation based on the determined associated threshold pulse fluence, wherein the relationship defines a decreasing threshold pulse fluence for a decreasing pulse duration in the range between 200 fs and smaller.

The relationship may further define the threshold pulse fluence as a value of at most 1.80 Jcm⁻², e.g., at most 1.50 Jcm⁻² or at most 1.30 Jcm⁻² or at most 1.10 Jcm⁻² or at most 0.90 Jcm⁻² or at most 0.70 Jcm⁻² or at most 0.50 Jcm⁻², for a pulse duration of 300 fs or smaller. In addition or as an alternative, the relationship may define the threshold pulse fluence as a value in the range from 0.80 Jcm⁻² to 1.50 Jcm⁻², e.g., in the range from 0.80 Jcm⁻² to 0.95 Jcm⁻² or from 0.95 Jcm⁻² to 1.05 Jcm⁻² or from 1.05 Jcm⁻² to 1.30 Jcm⁻² or from 1.30 Jcm⁻² to 1.50 Jcm⁻², for a pulse duration of 200 fs. In addition or as an alternative, the relationship may define the threshold pulse fluence as a value in the range from 0.20 Jcm⁻² to 0.50 Jcm⁻², e.g. in the range from 0.20 Jcm⁻² to 0.35 Jcm⁻² or from 0.35 Jcm⁻² to 0.50 Jcm⁻², for a pulse duration of 10 fs.

According to a third aspect a laser apparatus is described, the laser apparatus comprising a source of a beam of ultrashort-pulsed laser radiation, a set of components for guiding and shaping the beam in time and space, a control unit storing data representative of a relationship between a threshold pulse energy required for causing irreversible damage in a material and a pulse duration, the relationship allowing to obtain a threshold pulse energy for each of a plurality of pulse durations, the plurality of pulse durations including one or more pulse durations in a range between 200 fs and smaller, wherein the relationship defines a decreasing threshold pulse energy for a decreasing pulse duration in the range between 200 fs and smaller, wherein the control unit is configured to determine for a given pulse duration in the range between 200 fs and smaller an associated threshold pulse energy based on the stored data and to determine a target pulse energy for the beam based on the determined associated threshold pulse energy.

The control unit may be configured to output a visual representation of the determined target pulse energy on an output device. The output device may be a remote device or may be integral with the laser apparatus. In addition or as an alternative, the control unit may be configured to set the determined target pulse energy for the beam automatically.

The relationship may represent a decrease of the threshold pulse energy substantially as a function of the cubic root of the pulse duration. In addition or as an alternative, the relationship may define the threshold pulse energy as a value of at most 0.35 µJ, e.g., at most 0.30 µJ or at most 0.25 µJ or at most 0.20 µJ or at most 0.15 µJ, for a pulse duration of 300 fs or smaller. In addition or as an alternative, the relationship may define the threshold pulse energy as a value in the range from 0.15 µJ to 0.25 µJ, e.g., in the range from 0.18 µJ to 0.22 µJ, for a pulse duration of 200 fs. In addition or as an alternative, the relationship may define the threshold pulse energy as a value in the range from 0.05 µJ to 0.10 µJ, e.g., in the range from 0.06 µJ to 0.08 µJ, for a pulse duration of 10 fs.

The beam may be a Gaussian beam having an M² parameter of no more than 1.15 or 1.1.

Further details, objects and advantages of the invention become apparent from the following description and drawings.

### Brief Description of the Drawings

The invention is illustrated with reference to the following diagrams.
- Fig. 1: is a schematic representation of an embodiment of a method for determining threshold pulse energies for individual pulse durations according to the present invention;
- Fig. 2: is a schematic representation of an embodiment of a method for determining threshold pulse energies for a range of pulse durations according to the present invention;
- Fig. 3: is a flowchart of an embodiment of a method for energy setting of pulsed, focused laser radiation according to the present invention;
- Fig. 4: is a flowchart of an alternative embodiment of a method for energy setting of pulsed, focused laser radiation according to the present invention; and
- Fig. 5: is a schematic representation of an embodiment of a laser apparatus according to the present invention.

### Detailed Description

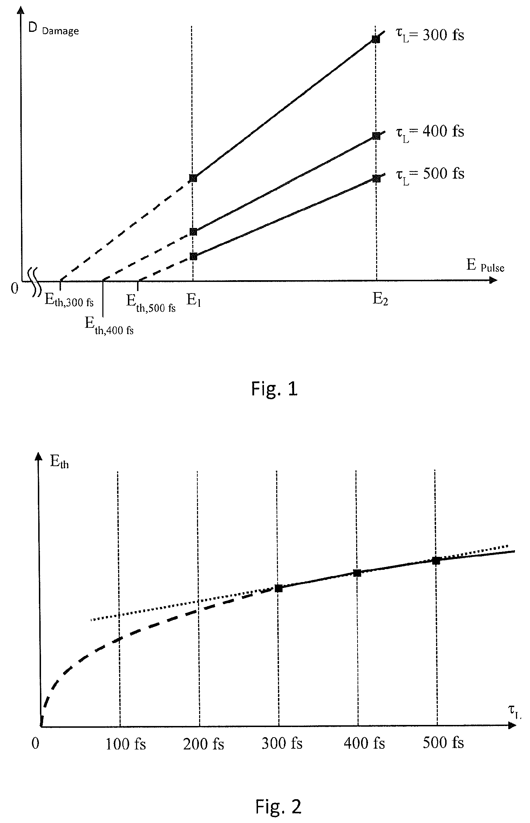

Fig. 1 illustrates schematically an embodiment of a method for determining, for particular pulse durations and for an arbitrary target material, a threshold pulse energy required to cause irreversible damage in the target material. In the example of Fig. 1, the pulse durations T_{L} are chosen as 300 fs, 400 fs and 500 fs, but the described method can also be applied to any other set of pulse lengths.

As shown in the diagram of Fig. 1, for any chosen pulse length a damage of finite size, D_{Damage}, will occur in the target material as soon as a pulse length-dependent threshold energy, Eₜₕ, is reached or exceeded. The diagram further shows that, for any given pulse length T_{L}, the size D_{Damage} of a damage site caused in the target material will increase with the pulse energy. From a comparison of the three curves it can be seen that a similar extent of damage as caused by a pulse of 500 fs and with energy E₂ can also be achieved with less energy, E₁, if that energy is concentrated in a shorter pulse of 300 fs. This agrees with the general assumption that, for similar effects, the use of shorter pulses allows for a smaller amount of energy transferred.

Since at the respective threshold pulse energies, E_{th,300}, E_{th,400}, E_{th,500}, the damage that is caused by a single pulse would be invisibly small, i.e., D_{Damage} = 0 and/or consist only in thermal changes in the material, the extent of the damage is determined for higher energies, E₁, E₂, where for a range of pulse durations the sizes of damage sites can be conveniently measured. As indicated by the dashed lines in Fig. 1, extrapolating that dependency for each pulse duration to a damage size of zero will subsequently yield values for the corresponding threshold energies.

Although the curves in Fig. 1 suggest a linear dependency of the damage size on the pulse energy, the dependency may often be better described by a non-linear relation. The exact relationship depends, for example, on the quantity that is chosen to describe the damage size, either by a length, an area or a volume. Moreover, whereas in the described example only single-pulse effects have been considered, the method may equally involve a detection of damage sizes for varying numbers of pulses applied at the same location (i.e. pulse bursts).

While conventional methods for determining threshold energies often rely on secondary effects that occur in connection with laser-induced optical breakdown, e.g., a rapid increase of plasma emission, the present method measures directly the intended effect of irreversible damage in the target material. In that way, threshold energies could be determined, experimentally and for different target materials, which differ from the results gained by other methods. The experiments suggest in particular that irreversible disruption can be achieved at lower threshold energies than is generally assumed. This, however, does not exclude the possibility that also in the present method the damage in the target material is at least partly caused by laser-induced optical breakdown.

Fig. 2 illustrates, schematically and for arbitrary pulse and material characteristics, a method for determining a relationship between the damage threshold pulse energy and pulse durations in ranges below and above 200 fs. In the range above 200 fs, the different threshold energies, Eₜₕ, according to Fig. 1 have been plotted for the corresponding pulse lengths T_{L} = 300 fs, 400 fs and 500 fs. As illustrated by the continuous curve in Fig. 2, interpolation allows one to establish the desired relationship in that range.

Based on the assumption that for ultrashort pulses disruption can be conceived as a mainly intensity-dependent process, which poses no lower boundary to the pulse length and, thus, to the threshold energy, it has been further assumed that the relationship expressed by the curve in Fig. 2 may start from the origin. In that case it is assumed that the threshold pulse energy is substantially a function of the cubic root of the pulse duration (E_{th(damage)} ∼ T^{1/3}). In connection with the measured data, and as illustrated by the dashed curve in Fig. 2, this allows an extrapolation of the curve beyond the measured range, also for pulse durations that are considerably shorter than 200 fs.

The resultant curve shows a continuous decrease of the threshold energy even towards shortest pulse lengths, and it is suitably described by a power function of the pulse length with an exponent smaller than 1. The curve thus implies that, if a low energy transfer into the target material is intended, pulse lengths can be reduced below the common usage, into the sub-200fs-range, while the threshold pulse energy decreases steadily for decreasing pulse durations. Once the described relationship has been established, it may be used for setting the pulse energy for various pulse durations in the range between 200 fs and less.

In addition to the above, or in a simplified embodiment which suffices without the assumption that the curve passes through the origin, the relationship may at least partially be determined on grounds of a linear approximation based on the measured data. This variant is exemplarily illustrated by the dotted curve in Fig. 2. Furthermore, since the described relationship relies on the assumption of a predominant intensity-dependency, it can be advantageous in alternative embodiments of the described method to ignore the pulse energy in favor of other energy-related beam parameters, such as the fluence per pulse.

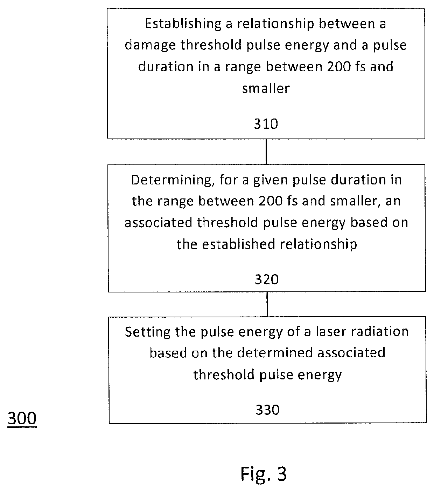

Fig. 3 is a flowchart of an exemplary embodiment of a method 300 for energy setting of pulsed, focused laser radiation according to the present invention. The method 300 may involve some or all of the above described procedures and results. In a first step 310, a relationship between a damage threshold pulse energy and a pulse duration in a range between 200 fs and less is established. This can be performed, for example, by the procedures described in connection with Figs. 1 and 2. Based on that relationship, and for a given pulse duration in the range between 200 fs and less, an associated threshold pulse energy is determined, step 320. Subsequently, in step 330, the pulse energy of a laser radiation is set based on the determined associated threshold pulse energy.

The method 300 thus allows to easily adjust the energy of laser radiation to a changed pulse length. In this way it becomes possible, for example, to vary for particular processes a pulse length of a laser while always maintaining an optimized pulse energy. If the pulse energy is set at a value larger than the determined threshold energy, the set value may in certain embodiments be, e.g., in a range between 1.5 and 5 times or between 1.5 and 4 times or between 1.8 and 3.5 times or between 2 and 4 times the determined threshold energy. According to other embodiments, the pulse energy of a laser apparatus used for processing a material may be set at at least 1.3 times or at least 1.5 times or at least 1.8 times or at least 2.0 times the determined threshold energy. As for an upper limit, the set pulse energy may be no more than 5 times or no more than 4.5 times or no more than 4 times or no more than 3.5 times or no more than 3.0 or no more than 2.5 times the determined threshold energy. In certain embodiments, the pulse energy may be set by a predetermined absolute amount, e.g., 0.05 µJ, 0.10 µJ or 0.20 µJ or 0.30 µJ or 0.40 µJ, above the determined threshold energy. In any of such cases, information about the threshold energy for a range of pulse lengths provides relevant means for optimizing the pulse energy accordingly.

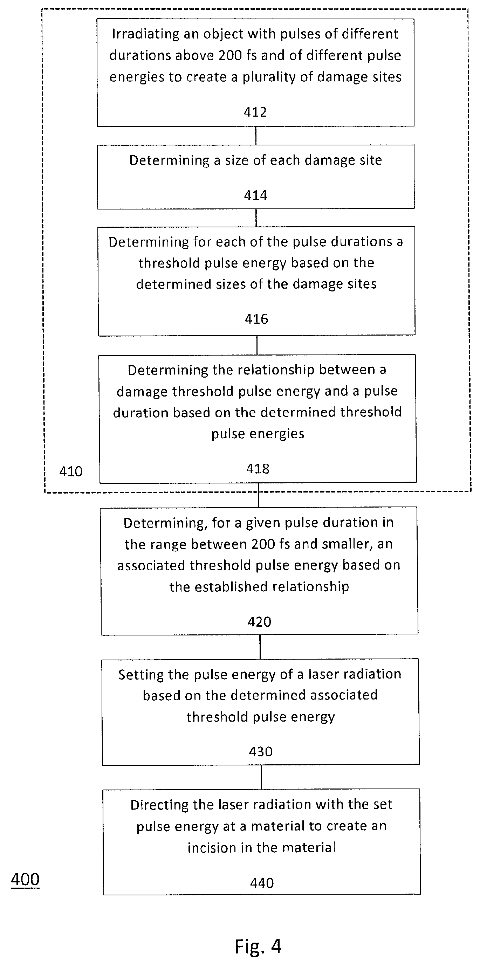

Fig. 4 shows a flowchart of an alternative embodiment of a method 400 for energy setting of pulsed, focused laser radiation according to the present invention. In the method 400 of Fig. 4, the step 410 for establishing a relationship between a damage threshold pulse energy and a pulse duration includes a plurality of sub-steps 412, 414, 416, 418. Analogous to the method 300 of Fig. 3, once that relationship has been established, it may be used to determine for a given pulse duration in the range between 200 fs and smaller an associated threshold pulse energy, step 420, and to set the pulse energy of a laser radiation based on the associated threshold pulse energy, step 430. Once the pulse energy has been set, the laser radiation can finally be directed at a target material to create an incision in the material, step 440.

In a first step 412 an object or sample of the material for which the described relationship is to be established is irradiated with different pulse energies and pulse durations above 200 fs such that measurable damage sites are produced in the object. The size of each damage site is then determined, step 414. Based on the determined sizes, a threshold pulse energy can be determined for each of the pulse durations, step 416. This may be performed by using any of the techniques described in connection with Fig. 1. Finally, based on the determined threshold energies, the relationship between a damage threshold pulse energy and a pulse duration is determined, step 418. This, again, can be done by using any of the techniques described in connection with Fig. 2.

An exact relationship between a damage threshold pulse energy and a pulse duration will depend also on numerous other conditions. These conditions include, most prominently, characteristics of the irradiated material and further beam parameters, such as the laser wavelength and the temporal and spatial profiles of the laser pulses. However, based on experimental data it turned out that for relevant applications the exponent of the power function that describes the sought relationship, as shown in Fig. 2, varies mainly between 0.3 and 0.36. It can therefore practically be approximated as a cubic root function of the pulse duration.

Moreover, the described method 300, 400 yields reliable results for different transparent non-biological and post mortem biological test materials such as Polymethylmethacrylat, PMMA, and animal eye tissue, and for beam characteristics in the most relevant ranges for established applications, e.g. when the diameter of the laser focus, i.e., the diameter of a beam cross-section that transmits ca. 86% of the pulse energy, is chosen smaller than 10 micrometers, e.g., smaller than 8 micrometers or smaller than 6 micrometers or smaller than 4 micrometers. For example, for pulse durations shorter than 300 fs, threshold energies between 0.05 and 0.35 micro-joules were determined. Within that range, the threshold energy for a pulse duration of 200 fs was determined to fall in a range from 0.15 to 0.30 micro-joules, and for a pulse duration of 10 fs, in a range from 0.05 to 0.1 micro-joules. It can therefore be expected, that the same characteristic cubic root-dependency of the threshold pulse energy on the pulse duration, and possibly also the same energy ranges, will apply in the case that the target material is human eye tissue.

As said before, alternative embodiments of the methods 300, 400 can be realized, in which other energy-related parameters, e.g., a fluence per pulse, instead of the pulse energy are considered. In such cases, too, the cubic-root dependency applies accordingly. For instance, with the same focus and material characteristics as in the previous example, and if applied to a threshold pulse fluence, the described method 300, 400 yields for pulse lengths less than 300 fs a threshold fluence between 0.2 and 1.80 Jcm⁻². More particular, the threshold fluence for a pulse duration of 200 fs was determined to fall in a range from 0.80 Jcm⁻² to 1.50 Jcm⁻², and for a pulse duration of 10 fs, in a range from 0.20 Jcm⁻² to 0.50 Jcm⁻².

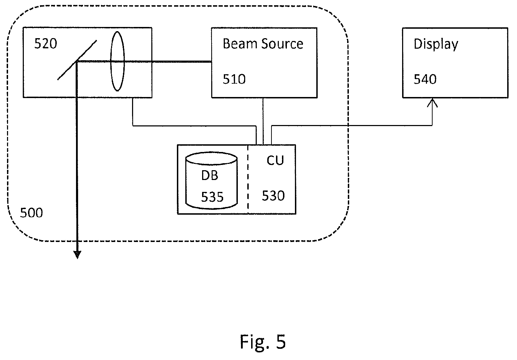

Fig. 5 shows an exemplary embodiment of a laser apparatus 500 according to the present invention. The laser apparatus 500 comprises a beam source 510, a set 520 of components for guiding and shaping the beam in time and space, and a control unit 530. The control unit 530 may comprise, or be connected to, a data base 535 such that the control unit 530 can access and process data that is stored by the data base 535. The laser apparatus 500 may further include a visual output device 540 and/or be adapted to output, by the control unit 530 and for visual representation, a signal to an external output device 540.

For simplicity, the beam source 510 and the set 520 of guiding and shaping components are shown as two distinct entities in Fig. 5. In alternative embodiments, however, the means for beam shaping and guiding may comprise a plurality of disjoint components in the laser apparatus 500; conversely, also the beam source 510 may comprise means for guiding and shaping a generated laser beam. It should therefore be understood that the beam source 510 together with the guiding and shaping means 520 define any technical arrangement as known in the art that is adapted to provide ultrashort-pulsed focused laser radiation, wherein at least a pulse length and a pulse energy or another energy-related parameter, such as a fluence per pulse, can be controlled. In view of the most relevant applications it is further preferable that the provided laser beam is a Gaussian beam with an M² parameter of no more than 1.15.

The control unit 530 may store and process data that is representative of a relationship between a pulse duration and a damage threshold pulse energy or a damage threshold for another energy-related pulse parameter, such as a threshold fluence, according to the present invention. For that purpose of data storage, the control unit 530 as shown in Fig. 5 comprises a storage device 535 that serves to host a data base. In alternative embodiments, the data base 535 may be arranged external to the control unit 530, provided that a functional connection between the control unit 530 and the data base 535 allows the control unit 530 to read and process data that is stored by the data base 535. Based on that stored data, the control unit 530 is configured to determine for a given pulse duration shorter than 200 fs an associated threshold pulse energy and, further, to determine an energy for pulses in a target beam based on the determined threshold value.

As shown in Fig. 5, the control unit 530 is further configured to output to the output device 540 a signal that indicates the determined target pulse energy. Graphical display of the determined target pulse energy thus enables a user of the laser apparatus to set a pulse energy according to the displayed information and dependent on the chosen pulse length. Alternatively, the control unit 530 may be adapted to set the determined pulse energy automatically. This can be achieved, for example, by control connections between the control unit and the beam source 510 and/or the set 520 of beam guiding and shaping means. This will allow a user of the laser apparatus 500 to arbitrarily vary the pulse length, whereas the laser apparatus 500 will automatically provide a corresponding pulse energy.

## Claims

1. A method (300; 400) for energy setting of pulsed, focused laser radiation, the method comprising:
- establishing (310; 410) a relationship between a threshold pulse energy required for causing irreversible damage in eye tissue and a pulse duration, the relationship allowing to obtain a threshold pulse energy for each of a plurality of pulse durations, the plurality of pulse durations including one or more pulse durations in a range between 200 fs and smaller;
- for a given pulse duration in the range between 200 fs and smaller, determining (320; 420) an associated threshold pulse energy based on the established relationship; and
- setting (330; 430) the pulse energy of the laser radiation based on the determined associated threshold pulse energy,
wherein the relationship defines a decreasing threshold pulse energy for a decreasing pulse duration in the range between 200 fs and smaller, **characterized in that** the establishing step (410) includes:
- irradiating (412), for each of a plurality of reference pulse durations above 200 fs, a non-biological material or post-mortem biological material with a series of pulses of the laser radiation to create a damage site for each pulse of the series, wherein the pulse energy is set differently for each pulse of the series;
- determining (414) a size of each damage site;
- determining (416) a reference threshold pulse energy for each of the plurality of reference pulse durations based on the determined sizes of the damage sites created at the respective reference pulse duration; and
- determining (418) the relationship based on the determined reference threshold pulse energies.

2. The method (300; 400) of claim 1, wherein the relationship represents a decrease of the threshold pulse energy substantially as a function of the cubic root of the pulse duration.

3. The method (300; 400) of claim 1 or 2, wherein the relationship defines the threshold pulse energy as a value of at most 0.35 µJ for a pulse duration of 300 fs or smaller, and/or wherein the relationship defines the threshold pulse energy as a value in the range from 0.15 µJ to 0.30 µJ for a pulse duration of 200 fs, and/or wherein the relationship defines the threshold pulse energy as a value in the range from 0.05 µJ to 0.10 µJ for a pulse duration of 10 fs.

4. The method (400) of any one of claims 1 to 3, wherein each reference threshold pulse energy is determined based on an extrapolation to zero size of the determined sizes of the damage sites created at the respective reference pulse duration.

5. The method (400) of any one of claims 1 to 4, wherein determining (418) the relationship includes determining a linear approximation of the threshold pulse energy in dependence on the pulse duration.

6. A method for fluence setting of pulsed, focused laser radiation, the method comprising:
- establishing a relationship between a threshold pulse fluence required for causing irreversible damage in eye tissue and a pulse duration, the relationship allowing to obtain a threshold pulse fluence for each of a plurality of pulse durations, the plurality of pulse durations including one or more pulse durations in a range between 200 fs and smaller;
- for a given pulse duration in the range between 200 fs and smaller, determining an associated threshold pulse fluence based on the established relationship; and
- setting the pulse fluence of the laser radiation based on the determined associated threshold pulse fluence,
wherein the relationship defines a decreasing threshold pulse fluence for a decreasing pulse duration in the range between 200 fs and smaller, **characterized in that** the establishing step includes:
- irradiating, for each of a plurality of reference pulse durations above 200 fs, a non-biological material or post-mortem biological material with a series of pulses of the laser radiation to create a damage site for each pulse of the series, wherein the pulse fluence is set differently for each pulse of the series;
- determining a size of each damage site;
- determining a reference threshold pulse fluence for each of the plurality of reference pulse durations based on the determined sizes of the damage sites created at the respective reference pulse duration; and
- determining the relationship based on the determined reference threshold pulse fluences..

7. The method (300; 400) of claim 6, wherein the relationship defines the threshold pulse fluence as a value of at most 1.80 Jcm⁻² for a pulse duration of 300 fs or smaller, and/or wherein the relationship defines the threshold pulse fluence as a value in the range from 0.80 Jcm⁻² to 1.50 Jcm⁻² for a pulse duration of 200 fs, and/or wherein the relationship defines the threshold pulse fluence as a value in the range from 0.20 Jcm⁻² to 0.50 Jcm⁻² for a pulse duration of 10 fs.

8. The method (300; 400) of any one of claims 1 to 7, wherein the relationship is established for a focus diameter of the laser radiation of no more than 10 µm or 7 µm or 5 µm, wherein the focus diameter represents the diameter of a pulse portion containing 86 % of the energy of a pulse of the radiation.

9. A laser apparatus (500) comprising:
- a source (510) of a beam of ultrashort-pulsed laser radiation;
- a set (520) of components for guiding and shaping the beam in time and space;
- a control unit (530, 535) storing data representative of a relationship between a threshold pulse energy required for causing irreversible damage in eye tissue and a pulse duration, the relationship allowing to obtain a threshold pulse energy for each of a plurality of pulse durations, the plurality of pulse durations including one or more pulse durations in a range between 200 fs and smaller, wherein the relationship defines a decreasing threshold pulse energy for a decreasing pulse duration in the range between 200 fs and smaller, wherein the control unit is configured to determine for a given pulse duration in the range between 200 fs and smaller an associated threshold pulse energy based on the stored data and to determine a target pulse energy for the beam based on the determined associated threshold pulse energy,
**characterized in that** the relationship defines the threshold pulse energy as a value of at most 0.35 µJ for a pulse duration of 300 fs or smaller, and wherein the relationship defines the threshold pulse energy as a value in the range from 0.15 µJ to 0.30 µJ for a pulse duration of 200 fs, and wherein the relationship defines the threshold pulse energy as a value in the range from 0.05 µJ to 0.1 µJ for a pulse duration of 10 fs.

10. The laser apparatus (500) of claim 9, wherein the control unit (530) is configured to output a visual representation of the determined target pulse energy on an output device (540).

11. The laser apparatus (500) of claim 9 or 10, wherein the control unit (530) is configured to set the determined target pulse energy for the beam automatically.

12. The laser apparatus (500) of any one of claims 9 to 11, wherein the relationship represents a decrease of the threshold pulse energy substantially as a function of the cubic root of the pulse duration.

13. The laser apparatus (500) of any one of claims 9 to 12, wherein the beam is a Gaussian beam having a M² parameter of no more than 1.15 or 1.1.

## Patentansprüche

1. Verfahren (300; 400) zum Energie-Einstellen von gepulster fokussierter Laserstrahlung, wobei das Verfahren umfasst:
- Erstellen (310; 410) eines Verhältnisses zwischen einer Schwellenwertpulsenergie, die erforderlich ist zum Verursachen irreversibler Beschädigung in Augengewebe, und einer Pulsdauer, wobei das Verhältnis gestattet, eine Schwellenwertpulsenergie für jede aus einer Mehrzahl von Pulsdauern zu erhalten, wobei die Mehrzahl von Pulsdauern eine oder mehr Pulsdauern in einem Bereich zwischen 200 fs und kleiner enthalten;
- für eine gegebene Pulsdauer in dem Bereich zwischen 200 fs und kleiner, Bestimmen (320; 420) einer zugeordneten Schwellenwertpulsenergie auf der Grundlage der erstellten Beziehung; und
- Einstellen (330; 430) der Pulsenergie der Laserstrahlung auf der Grundlage der bestimmten zugeordneten Schwellenwertpulsenergie, wobei das Verhältnis eine abnehmende Schwellenwertpulsenergie für eine abnehmende Pulsdauer in dem Bereich zwischen 200 fs und kleiner definiert,
**dadurch gekennzeichnet, dass**
der Erstellensschritt (410) enthält:
- Bestrahlen (412), für jede aus einer Mehrzahl von Referenzpulsdauern oberhalb von 200 fs, eines nicht-biologischen Materials oder eines post-mortem biologischen Materials mit einer Serie von Pulsen der Laserstrahlung, um für jeden Puls der Serie eine Beschädigungsstelle zu erzeugen, wobei die Pulsenergie für jeden Puls der Serie verschieden eingestellt ist;
- Bestimmen (414) einer Größe jeder Beschädigungsstelle;
- Bestimmen (416) einer Referenzschwellenwertpulsenergie für jede aus der Mehrzahl von Referenzpulsdauern auf der Grundlage der bestimmten Größen der Beschädigungsstellen, die bei der jeweiligen Referenzpulsdauer erzeugt wurden; und
- Bestimmen (418) des Verhältnisses auf der Grundlage der bestimmten Referenzschwellenwertpulsenergien.

2. Verfahren (300; 400) nach Anspruch 1, wobei das Verhältnis eine Abnahme der Schwellenwertpulsenergie im Wesentlichen als eine Funktion der Kubikwurzel der Pulsdauer darstellt.

3. Verfahren (300; 400) nach Anspruch 1 oder 2, wobei das Verhältnis die Schwellenwertpulsenergie als einen Wert von höchstens 0,35 µJ für eine Pulsdauer von 300 fs oder kleiner definiert und/oder wobei das Verhältnis die Schwellenwertpulsenergie als einen Wert in dem Bereich von 0,15 µJ bis 0,30 µJ für eine Pulsdauer von 200 fs definiert und/oder wobei das Verhältnis die Schwellenwertpulsenergie als einen Wert in dem Bereich von 0,05 µJ bis 0,10 µJ für eine Pulsdauer von 10 fs definiert.

4. Verfahren (400) nach einem der Ansprüche 1 bis 3, wobei jede Referenzschwellenwertpulsenergie auf der Grundlage einer Extrapolation zur Größe 0 der bestimmten Größen der Beschädigungsstellen, die bei der jeweiligen Referenzpulsdauer erzeugt wurden, bestimmt wird.

5. Verfahren (400) nach einem der Ansprüche 1 bis 4, wobei das Bestimmen (418) des Verhältnisses enthält Bestimmen einer linearen Approximation der Schwellenwertpulsenergie in Abhängigkeit von der Pulsdauer.

6. Verfahren zum Fluenz-Einstellen von gepulster fokussierter Laserstrahlung, wobei das Verfahren umfasst:
- Erstellen eines Verhältnisses zwischen einer Schwellenwertpulsfluenz, die erforderlich ist zum Verursachen irreversibler Beschädigung in Augengewebe, und einer Pulsdauer, wobei das Verhältnis gestattet, eine Schwellenwertpulsfluenz für jede aus einer Mehrzahl von Pulsdauern zu erhalten, wobei die Mehrzahl von Pulsdauern eine oder mehr Pulsdauern in einem Bereich zwischen 200 fs und kleiner enthält;
- für eine gegebene Pulsdauer in dem Bereich zwischen 200 fs und kleiner, Bestimmen einer zugeordneten Schwellenwertpulsfluenz auf der Grundlage des erstellten Verhältnisses; und
- Einstellen der Pulsfluenz der Laserstrahlung auf der Grundlage der bestimmten zugeordneten Schwellenwertpulsfluenz,
wobei das Verhältnis eine abnehmende Schwellenwertpulsfluenz für eine abnehmende Pulsdauer in dem Bereich zwischen 200 fs und kleiner definiert, **dadurch gekennzeichnet, dass**
der Erstellensschritt enthält:
- Bestrahlen, für jede aus einer Mehrzahl von Referenzpulsdauern oberhalb von 200 fs, eines nicht-biologischen Materials oder eines post-mortem biologischen Materials mit einer Serie von Pulsen der Laserstrahlung, um eine Beschädigungsstelle für jeden Puls aus der Serie zu erzeugen, wobei die Pulsfluenz für jeden Puls der Serie verschieden eingestellt ist;
- Bestimmen einer Größe jeder Beschädigungsstelle;
- Bestimmen einer Referenzschwellenwertpulsfluenz für jede aus der Mehrzahl von Referenzpulsdauern auf der Grundlage der bestimmten Größen der Beschädigungsstellen, die bei der jeweiligen Referenzpulsdauer erzeugt wurden; und
- Bestimmen des Verhältnisses auf der Grundlage der bestimmten Referenzschwellenwertpulsfluenzen.

7. Verfahren (300; 400) nach Anspruch 6, wobei das Verhältnis die Schwellenwertpulsfluenz als einen Wert von höchstens 1,80 Jcm⁻² für eine Pulsdauer von 300 fs oder kleiner definiert, und/oder wobei das Verhältnis die Schwellenwertpulsfluenz als einen Wert in dem Bereich von 0,80 Jcm⁻² bis 1,50 Jcm⁻² für eine Pulsdauer von 200 fs definiert, und/oder wobei das Verhältnis die Schwellenwertpulsfluenz als einen Wert in dem Bereich von 0,20 Jcm⁻² bis 0,50 Jcm⁻² für eine Pulsdauer von 10 fs definiert.

8. Verfahren (300; 400) nach einem der Ansprüche 1 bis 7, wobei das Verhältnis erstellt wird für einen Fokusdurchmesser der Laserstrahlung von nicht mehr als 10 µm oder 7 µm oder 5 µm, wobei der Fokusdurchmesser den Durchmesser eines Pulsanteils darstellt, der 86 % der Energie eines Pulses der Strahlung enthält.

9. Laservorrichtung (500) umfassend:
- eine Quelle (510) eines Strahls von ultrakurz-gepulster Laserstrahlung;
- einen Satz (520) von Komponenten zum Führen und Formen des Strahls in Zeit und Raum;
- eine Steuerungseinheit (530, 535), die Daten speichert, die repräsentativ sind für ein Verhältnis zwischen einer Schwellenwertpulsenergie, die erforderlich ist zum Verursachen Irreversibler Beschädigung in Augengewebe, und einer Pulsdauer, wobei das Verhältnis gestattet, eine Schwellenwertpulsenergie für jede aus einer Mehrzahl von Pulsdauern zu erhalten, wobei die Mehrzahl von Pulsdauern eine oder mehr Pulsdauern in einem Bereich zwischen 200 fs und kleiner enthält, wobei das Verhältnis eine abnehmende Schwellenwertpulsenergie für eine abnehmende Pulsdauer in dem Bereich zwischen 200 fs und kleiner definiert, wobei die Steuerungseinheit dazu ausgebildet ist, für eine gegebene Pulsdauer in dem Bereich zwischen 200 fs und kleiner eine zugeordnete Schwellenwertpulsenergie auf der Grundlage der gespeicherten Daten zu bestimmen und eine Zielpulsenergie für den Strahl auf der Grundlage der bestimmten zugeordneten Schwellenwertpulsenergie zu bestimmen,
**dadurch gekennzeichnet, dass**
das Verhältnis die Schwellenwertpulsenergie als einen Wert von höchstens 0,35 µJ für eine Pulsdauer von 300 fs oder kleiner definiert und wobei das Verhältnis die Schwellenwertpulsenergie als einen Wert in dem Bereich von 0,15 µJ bis 0,30 µJ für eine Pulsdauer von 200 fs definiert, und wobei das Verhältnis die Schwellenwertpulsenergie als einen Wert in dem Bereich 0,05 µJ bis 0,1 µJ für eine Pulsdauer von 10 fs definiert.

10. Laservorrichtung (500) nach Anspruch 9, wobei die Steuerungseinheit (530) dazu ausgebildet ist, eine visuelle Darstellung der bestimmten Zielpulsenergie an einem Ausgabegerät (540) auszugeben.

11. Laservorrichtung (500) nach Anspruch 9 oder 10, wobei die Steuerungseinheit (530) dazu ausgebildet ist, die bestimmte Zielpulsenergie des Strahls automatisch einzustellen.

12. Laservorrichtung (500) nach einem der Ansprüche 9 bis 11, wobei das Verhältnis eine Abnahme der Schwellenwertpulsenergie im Wesentlichen als eine Funktion der Kubikwurzel der Pulsdauer darstellt.

13. Laservorrichtung (500) nach einem der Ansprüche 9 bis 12, wobei der Strahl ein Gauß-Strahl ist, der einen M²-Parameter von nicht mehr als 1,15 oder 1,1 aufweist.

## Revendications

1. Procédé (300 ; 400) de réglage d'énergie de rayonnement laser pulsé et focalisé, le procédé comprenant :
- l'établissement (310 ; 410) d'un rapport entre une énergie d'impulsion de seuil nécessaire pour provoquer des dommages irréversibles dans le tissu oculaire et une durée d'impulsion, le rapport permettant d'obtenir une énergie d'impulsion de seuil pour chacune d'une pluralité de durées d'impulsion, la pluralité de durées d'impulsion comprenant une ou plusieurs durées d'impulsion dans une plage comprise entre 200 fs et inférieure ;
- pendant une durée d'impulsion donnée dans la plage comprise entre 200 fs et inférieure, la détermination (320 ; 420) d'une énergie d'impulsion de seuil associée sur la base du rapport établi ; et
- la fixation (330 ; 430) de l'énergie d'impulsion du rayonnement laser sur la base de l'énergie d'impulsion de seuil associée déterminée,
dans lequel le rapport définit une énergie d'impulsion de seuil décroissante pendant une durée d'impulsion décroissante dans la plage comprise entre 200 fs et inférieure,
**caractérisé en ce que**
l'étape d'établissement (410) comprend :
- l'irradiation (412), pour chacune d'une pluralité de durées d'impulsion de référence au-dessus de 200 fs, d'un matériau non biologique ou d'un matériau biologique post-mortem avec une série d'impulsions du rayonnement laser pour créer un site endommagé pour chaque impulsion de la série, dans lequel l'énergie d'impulsion est définie différemment pour chaque impulsion de la série ;
- la détermination (414) d'une taille de chaque site endommagé ;
- la détermination (416) d'une énergie d'impulsion de seuil de référence pour chacune de la pluralité de durées d'impulsion de référence sur la base des tailles déterminées des sites endommagés créés au niveau de la durée d'impulsion de référence respective ; et
- la détermination (418) du rapport sur la base des énergies d'impulsion de seuil de référence déterminées.

2. Procédé (300 ; 400) selon la revendication 1, dans lequel le rapport représente une diminution de l'énergie d'impulsion de seuil sensiblement en fonction de la racine cubique de la durée d'impulsion.

3. Procédé (300 ; 400) selon la revendication 1 ou 2, dans lequel le rapport définit l'énergie d'impulsion de seuil en tant que valeur d'au maximum 0,35 µJ pendant une durée d'impulsion de 300 fs ou inférieure, et/ou dans lequel le rapport définit l'énergie d'impulsion de seuil en tant que valeur dans la plage allant de 0,15 µJ à 0,30 µJ pendant une durée d'impulsion de 200 fs, et/ou dans lequel le rapport définit l'énergie d'impulsion de seuil en tant que valeur dans la plage allant de 0,05 µJ à 0,10 µJ pendant une durée d'impulsion de 10 fs.

4. Procédé (400) selon l'une quelconque des revendications 1 à 3, dans lequel chaque énergie d'impulsion de seuil de référence est déterminée sur la base d'une extrapolation de taille nulle parmi les tailles déterminées des sites endommagés créés au niveau de la durée d'impulsion de référence respective.

5. Procédé (400) selon l'une quelconque des revendications 1 à 4, dans lequel la détermination (418) du rapport consiste à déterminer une approximation linéaire de l'énergie d'impulsion de seuil en fonction de la durée d'impulsion.

6. Procédé de réglage de fluence de rayonnement laser pulsé et focalisé, le procédé comprenant :
- l'établissement d'un rapport entre une fluence d'impulsion de seuil nécessaire pour provoquer des dommages irréversibles dans le tissu oculaire et une durée d'impulsion, le rapport permettant d'obtenir une fluence d'impulsion de seuil pour chacune d'une pluralité de durées d'impulsion, la pluralité de durées d'impulsion comprenant une ou plusieurs durées d'impulsion dans une plage comprise entre 200 fs et inférieure ;
- pendant une durée d'impulsion donnée dans la plage comprise entre 200 fs et inférieure, la détermination d'une fluence d'impulsion de seuil associée sur la base du rapport établi ; et
- la fixation de la fluence d'impulsion du rayonnement laser sur la base de la fluence d'impulsion de seuil associée déterminée,
dans lequel le rapport définit une fluence d'impulsion de seuil décroissante pendant une durée d'impulsion décroissante dans la plage comprise entre 200 fs et inférieure,
**caractérisé en ce que**
l'étape d'établissement comprend :
- l'irradiation, pour chacune d'une pluralité de durées d'impulsion de référence au-dessus de 200 fs, d'un matériau non biologique ou d'un matériau biologique post-mortem avec une série d'impulsions du rayonnement laser pour créer un site endommagé pour chaque impulsion de la série, dans lequel la fluence d'impulsion est définie différemment pour chaque impulsion de la série ;
- la détermination d'une taille de chaque site endommagé ;
- la détermination d'une fluence d'impulsion de seuil de référence pour chacune de la pluralité de durées d'impulsion de référence sur la base des tailles déterminées des sites endommagés créés au niveau de la durée d'impulsion de référence respective ; et
- la détermination du rapport sur la base des fluences d'impulsion de seuil de référence déterminées.

7. Procédé (300 ; 400) selon la revendication 6, dans lequel le rapport définit la fluence d'impulsion de seuil en tant que valeur d'au maximum 1,80 Jcm⁻² pendant une durée d'impulsion de 300 fs ou inférieure, et/ou dans lequel le rapport définit la fluence d'impulsion de seuil en tant que valeur dans la plage allant de 0,80 Jcm⁻² à 1,50 Jcm⁻² pendant une durée d'impulsion de 200 fs, et/ou dans lequel le rapport définit la fluence d'impulsion de seuil en tant que valeur dans la plage allant de 0,20 Jcm⁻² à 0,50 Jcm⁻² pendant une durée d'impulsion de 10 fs.

8. Procédé (300 ; 400) selon l'une quelconque des revendications 1 à 7, dans lequel le rapport est établi pour un diamètre de foyer du rayonnement laser de pas plus de 10 µm, 7 µm ou 5 µm, dans lequel le diamètre de foyer représente le diamètre d'une partie d'impulsion contenant 86 % de l'énergie d'une impulsion du rayonnement.

9. Appareil laser (500) comprenant :
- une source (510) d'un faisceau de rayonnement laser pulsé ultracourt ;
- un ensemble (520) de composants permettant de guider et de mettre en forme le faisceau dans le temps et l'espace ;
- une unité de commande (530, 535) stockant des données représentant un rapport entre une énergie d'impulsion de seuil nécessaire pour provoquer des dommages irréversibles dans le tissu oculaire et une durée d'impulsion, le rapport permettant d'obtenir une énergie d'impulsion de seuil pour chacune d'une pluralité de durées d'impulsion, la pluralité de durées d'impulsion comprenant une ou plusieurs durées d'impulsion dans une plage comprise entre 200 fs et inférieure, dans lequel le rapport définit une énergie d'impulsion de seuil décroissante pendant une durée d'impulsion décroissante dans la plage comprise entre 200 fs et inférieure, dans lequel l'unité de commande est configurée pour déterminer une durée d'impulsion donnée dans la plage comprise entre 200 fs et inférieure, une énergie d'impulsion de seuil associée sur la base des données stockées, et pour déterminer une énergie d'impulsion cible pour le faisceau sur la base de l'énergie d'impulsion de seuil associée déterminée,
**caractérisé en ce que**
le rapport définit l'énergie d'impulsion de seuil en tant que valeur d'au maximum 0,35 µJ pendant une durée d'impulsion de 300 fs ou inférieure, et dans lequel le rapport définit l'énergie d'impulsion de seuil en tant que valeur dans la plage allant de 0,15 µJ à 0,30 µJ pendant une durée d'impulsion de 200 fs, et dans lequel le rapport définit l'énergie d'impulsion de seuil en tant que valeur dans la plage allant de 0,05 µJ à 0,1 µJ pendant une durée d'impulsion de 10 fs.

10. Appareil laser (500) selon la revendication 9, dans lequel l'unité de commande (530) est configurée pour délivrer en sortie une représentation visuelle de l'énergie d'impulsion cible déterminée sur un dispositif de sortie (540).

11. Appareil laser (500) selon la revendication 9 ou 10, dans lequel l'unité de commande (530) est configurée pour fixer l'énergie d'impulsion cible déterminée pour le faisceau automatiquement.

12. Appareil laser (500) selon l'une quelconque des revendications 9 à 11, dans lequel le rapport représente une diminution de l'énergie d'impulsion de seuil sensiblement en fonction de la racine cubique de la durée d'impulsion.

13. Appareil laser (500) selon l'une quelconque des revendications 9 à 12, dans lequel le faisceau est un faisceau gaussien ayant un paramètre M² d'au maximum 1,15 ou 1,1.
